# EUROPEAN PATENT SPECIFICATION

(11) **EP 3 233 885 B1**
(45) Date of publication and mention of the grant of the patent: **04.11.2020**
(21) Application number: 15823187.8
(22) Date of filing: 16.12.2015
(51) Int. Cl.: C07K 1/30, C12N 9/42

(54) **METHOD FOR CONCENTRATING PROTEINS**
VERFAHREN ZUR KONZENTRIERUNG VON PROTEINEN
PROCÉDÉ POUR CONCENTRER DES PROTÉINES

(30) Priority: 18.12.2014 FI 20146113
(43) Date of publication of application: 25.10.2017
(73) Proprietor: AB Enzymes Oy, 05200 Rajamäki (FI)
(72) Inventor: PALMUNEN, Katja, 05200 Rajamäki (FI); PERKKALAINEN, Mirkka, 01730 Vantaa (FI)
(74) Representative: Espatent Oy
(86) International application number: PCT/FI2015/050890
(87) International publication number: WO 2016/097484

(56) References cited:
- WO-A1-97/15660
- JOHANSSON G ET AL: "Concentration and purification of beta-glucosidase from Aspergillus niger by using aqueous two-phase partitioning", JOURNAL OF CHROMATOGRAPHY B: BIOMEDICAL SCIENCES & APPLICATIONS, ELSEVIER, AMSTERDAM, NL, vol. 711, no. 1-2, 26 June 1998 (1998-06-26), pages 161-172, XP004129433, ISSN: 1570-0232, DOI: 10.1016/S0378-4347(97)00601-4

## Description

### FIELD OF THE INVENTION

The present description is related to the field of enzyme production. More specifically, it discloses a method for rapidly purifying, concentrating and harvesting cellulases from spent fermentation broth and providing them in very high concentration formulations wherein they retain their enzymatic activity and stability for a long time. The method is simple, very effective and cheap, and it is compatible with industrial requirements.

### BACKGROUND

Industrial enzymes are usually provided as liquids or dry powders of cell lysates or mixtures of proteins secreted into the fermentation broth. Even though the enzyme of interest may be the primary enzyme component of the enzyme preparation, in many applications the other residual enzyme activities as well as impurities present in the enzyme preparation are harmful in many ways, e.g. they may have an adverse effect on performance, quality or stability of the enzyme preparation. Even though highly pure enzyme preparations would be desirable and impurities can often be removed, in industrial scale enzyme purification is impractical because of the high costs and poor yield. Further, even when enzymes are purified in industrial scale, the resulting preparation is often a complex mixture of proteins and other macromolecules which has to be concentrated for further use and storage.

Crystalline enzymes would be desirable for industrial use. In a crystalline state an enzyme is in a highly pure state because an enzyme crystal contains only a single molecular species of the enzyme. Further, in a crystalline state the enzyme is in a very high concentration. Thus, crystallization of enzymes would provide an effective method to purify enzymes and provide them in a very high concentration. However, crystallization of enzymes is not straightforward and it often requires a lot of empirical work. Generally, industrial enzymes cannot be easily crystallized: even minor amounts of impurities or fragments of the enzyme can prevent crystallization and often crystallization of the desired enzyme is completely impossible. Usually initiation of crystallization requires that the enzyme is both purified from impurities and concentrated into a high concentration. A lot of empirical work is required to find crystallization conditions for a certain enzyme molecule and to optimize those conditions further.

Typically a high salt concentration is required for enzyme crystallization especially to achieve high yield that is essential for an industrial process. However, high salt concentrations are not economically or environmentally acceptable because of increased chemical cost and environmental load. Further, crystallization attempts often fail and may lead into precipitated enzyme preparations in the form of suspensions that are difficult to process further and may have low enzyme activity. Thus, crystallization is generally too impractical method for purifying and concentrating industrial enzymes.

Cellulase enzymes consist of a catalytic domain, or a core, responsible for the cellulose degrading reaction. In addition to the catalytic domain, the cellulase molecule may comprise one or more cellulose binding moieties (CBM; also called cellulose binding domains, CBD) which mediate binding of the enzyme to the substrate. The catalytic domain attached to the cellulose binding moiety is preferably joined by a linker peptide, which is sufficiently long and flexible to allow efficient orientation and operation of both domains. CBMs contribute significantly to the activity of cellulases against cellulose and their presence in industrial cellulase preparations is preferred.

Crystallization of cellulases having a CBM is not known in prior art. Attempts have been made to provide crystalline cellulases without CBM. US6190898 describes a method for crystallizing ultrafiltrated EGIII cellulase without CBM by adding salt. The disclosure mentions that CBM provides unfavorable crystallization kinetics and prevents crystallization of EGIII if present in the enzyme. The structure of the CBM from Trichoderma reesei CBH I (cellobiohydrolase I) has been solved, but no structure of an intact multidomain cellulose, consisting of the catalytic core, linker and CBM, has been determined (Kleywegt et al., 1997).The crystallization of intact fungal cellulases has not been successful (Koivula et al. 1998), the linkers have so far prevented the attempts to crystallize an intact cellulase with all its domains (Maurer 1997).

WO 97/15660 discloses a method of crystallising a modified cellulase lacking a cellulose binding domain.

Thus, in enzyme industry there is a need to produce highly pure and concentrated enzyme compositions in an economical manner. In particular, there is a need for a concentrated composition of pure cellulase with CBM.

### SUMMARY

Accordingly, it is an object of the invention to provide an improved simple and effective method of producing highly pure cellulases in a concentrated formulation. Further, it is an object of the invention to provide a method for crystallizing cellulase with a CBM.

These and other objects of the invention have been attained by the provision of the present method wherein cellulases can be purified and concentrated directly from an impure aqueous medium by adjusting pH. The crystal yield can be further increased to a level that is preferred in an industrial process by adding a low concentration of polyethylene glycol (PEG) after the crystallization has initiated.

According to the first aspect of the invention there is provided a method for concentrating a cellulase from spent fermentation broth comprising the sequence of steps:
a. providing the cellulase in the spent fermentation broth in a soluble form;
b. controlling cellulase solubility by adjusting protein concentration; adjusting the pH value to a pH value selected between between pH 3 and 6, to provide the cellulase in a solid phase, and adding polyethylene glycol to increase the amount of cellulase in the solid phase; and
c. harvesting the cellulase from the solid phase.

In the present method solubility of the enzyme is controlled by adjusting pH and adding polyethylene glycol which results in precipitation of the enzyme in a solid phase at high yield. The solid cellulase can be easily harvested from the fermentation broth. Suitable methods to harvest the cellulase from the fermentation broth include filtration, sedimentation, and centrifugation.

The cellulase harvested in the solid phase may encompass the cellulase in the form of a precipitate, a gel, a spherulite, a microcrystal, and/or a crystal. Precipitate, spherulites, microcrystals and crystals are preferred. The harvested cellulase in the solid phase may comprise or consist of cellulase crystals. The cellulase crystals may comprise majority of the harvested cellulase. As is understood in the art, a protein in a solid phase, such as a crystalline protein, is in a solubility equilibrium in the aqueous medium and, accordingly, in a concentrated protein formulation the protein may be present simultaneously in more than one phase. Thus, a substantially crystalline formulation of an enzyme may comprise the enzyme also in a precipitated phase as well as in a soluble phase.

The harvested solid cellulase can be used as semifinal product for purified liquid formulation by dissolving it at conditions wherein enzyme solubility is high enough.

The present method is effective even when the aqueous medium is a fermentation broth. Thus, the present method makes it possible to achieve high quality final products having standardized and clearly defined formulation background into the market.

The present method is advantageous in that it synergistically combines purification and concentrating steps even directly from a fermentation broth. The present invention effectively uses a single step method which produces the cellulase in a form which is easy to store, transport and use. Concentrated cellulase suspension according to the invention is safer and easier to handle as a semifinal product compared to a dusty enzyme powder obtained using prior art methods. Further, as both the purification and the concentrating steps are combined in the present method, a very high yield of cellulase can be achieved. Previous methods have used separate process steps for purification and concentrating, thus resulting into a lower yield because of loss in recovery between the steps.

Further, the present invention provides for the first time an industrially applicable method that can produce crystals of a cellulase which contains CBM. Producing cellulase with CBM helps to deliver the catalytic domain of the cellulase into close contact with cellulose for efficient hydrolysis. Thus, providing the present highly pure and concentrated cellulase with the CBM domain makes the cellulase more efficient in hydrolyzing cellulose and, consequently, less enzyme is required in industrial processes wherein the cellulase activity is used.

Additionally, the present method is advantageous because it makes it possible to formulate cellulase compositions of very high concentration. Further, when cellulase crystals are harvested as a crystal suspension, the suspension is easier and safer to handle and transport in post-processing compared to dry enzyme powders. Even if the cellulase crystal suspension is dried, less solvent needs to be removed from the suspension because of the high concentration of the cellulase already present in the crystals, resulting in a more efficient and economical formulating process.

Another advantage may be that the resulting cellulase crystals are stabile and remain crystalline even outside the high yield crystallization conditions, e.g. when polyethylene glycol is removed. This is advantageous in particular in applications where presence of polyethylene glycol should be avoided.

According to the second aspect of the invention there is provided a composition comprising at least partially crystallised cellulase produced according to the first aspect, wherein the composition is in a liquid or in a solid form.

The composition may comprise at least partially crystallised cellulase produced according to the first aspect, wherein the composition is in a liquid or in a solid form

The composition may have improved quality and physical stability as a liquid or a solid form at a desired high activity level. The composition may also comprise various compositions because the cellulase produced according to the first aspect is stabile in various chemical and physical conditions.

According to the third aspect there is provided a use of the composition according to the second aspect to degrade cellulose in biomass processing, biofuel, starch, textile, detergent, pulp and paper, food, baking, feed or beverage industry.

According to the fourth aspect there is provided a composition obtained by drying the cellulase produced according to the second aspect or the composition according to the third aspect.

The enzyme composition harvested using the present method shows good purification and formulation conditions as evidenced by the solubility diagrams. The solubility diagrams are utilized also as basic data for optimized and controlled industrial scale crystallization process based on the present method. The present method is simple, effective and inexpensive method for purification and concentration of cellulases.

### BRIEF DESCRIPTION OF THE FIGURES

Figure 1 is a diagram showing the solubility of crystals of Ma_Cel45A+CBM protein as function of pH.
Figure 2 is a diagram showing gel filtration result of washed crystals of Ma_Cel45A+CBM protein.
Figure 3 is a diagram showing the solubility of crystals of Ma_Cel45A+CBM protein as function of pH in Na-citrate buffer and Na-K-phosphate buffer.
Figure 4 is a diagram showing strong solubility decreasing effect of PEG when the solubility of crystals of Ma_Cel45A+CBM protein is presented as function of pH in Na-citrate buffer and PEG 3350.
Figure 5 is a diagram showing the solubility of crystals of Ma_Cel45A+CBM protein as function of PEG 8000 concentration.
Figure 6 is a diagram showing the solubility of washed crystals of Ma_Cel45A+CBM protein as function of PEG 8000 concentration.
Figure 7 is a picture showing the crystals of Ma_Cel45A+CBM protein from solubility test with 6 % (w/v) PEG 8000 at pH 4.3.
Figure 8 is a diagram showing the solubility of washed crystals of Ma_Cel45A+CBM protein as a function of ammonium sulphate concentration.
Figure 9 is a diagram showing the solubility of washed crystals of Ma_Cel45A+CBM protein in 15 % and 25 % (w/v) PEG 8000 as function of pH.
Figure 10 is a picture showing the crystals of Ma_Cel45A+CBM protein from solubility test with 25 % (w/v) PEG 8000 at pH 5.9.
Figure 11 is a diagram showing the solubility of washed crystals of Ma_Cel45A+CBM protein in 10 % (w/v) PEG 8000 as function of temperature.
Figure 12 is a picture showing seed crystals of Ma_Cel45A+CBM protein in clarified fermentation broth buffered to pH 3.4.
Figure 13 is a picture showing crystallization of Ma_Cel45A+CBM protein directly from the clarified fermentation broth at acidic pH by adding 3 % (w/v) PEG 8000.
Figure 14 is a picture showing spherulites in At_Cel45A+CBM fermentation broth sample achieved by hanging drop method.
Figure 15 is a picture showing purified core domain At_Cel45A without CBM crystallized by hanging drop method.

### DETAILED DESCRIPTION

The method may be carried out in the sequence a., b., and c.

The term cellulase refers herein to bacterial, plant and fungal exoglucanases, exocellobiohydrolases, endoglucanases, and beta-glucosidases.

In an embodiment the cellulase has a cellulose binding moiety, preferably a fungal family 1 carbohydrate binding moiety. In an embodiment the cellulase is a fusion protein wherein the structural elements responsible for the catalytic activity and the CBM part have a different origin. Thus, according to the present invention it is possible to produce a cellulase which has a CBM with a substrate binding affinity and/or specificity to a specific type of cellulose, such as amorphous or crystalline cellulose. In another embodiment the CBM has the same origin as the structural elements responsible for the catalytic activity, i.e. the cellulase has the CBM which it naturally has as a cellulase enzyme. The CBM can be either N-terminal or C-terminal.

Cellulose binding moiety, CBM, refers herein to a group of related peptides responsible in large part for the cellulose binding activity of the cellulase or derivative thereof. Cellulose binding moieties generally function by non-covalently binding the cellulase to cellulose, a cellulose derivative or other polysaccharide equivalent of cellulose. Cellulose binding moieties as defined herein attach the enzyme to cellulose in a manner which permits or facilitates hydrolysis of cellulose fibers. A cellulose binding moiety is a structural element of the cellulase enzyme tertiary structure which is distinct from the structural element which possesses substantially all catalytic cellulase activity. CBM may be linked to the cellulase catalytic core through a hinge or a linker region.

In an embodiment the present cellulase is a GH45 (Glycoside hydrolase family 45, see for example CAZy web site at http://www.cazy.org/) endoglucanase. In yet another embodiment the present cellulase is Cel45A (Haakana et al. 2004) selected from *Melanocarpus albomyces* endoglucanase with fungal family 1 carbohydrate-binding module, optionally produced in *Trichoderma reesei* (Ma_Cel45A+CBM); and *Acremonium thermophilum* endoglucanase with fungal family 1 carbohydrate-binding module, optionally produced in *Trichoderma reesei* (At_Cel45A+CBM) (WO2007071820).

In an embodiment is provided a method for concentrating a cellulase from spent fermentation broth, the method comprising the sequence of steps:
providing the cellulase in the spent fermentation broth in a soluble form;
adjusting the pH value to a pH value selected between pH 3 and 6, to provide and maintain cellulase in a solid phase;
adding polyethylene glycol to increase the amount of the cellulase in the solid phase; and
harvesting the cellulase from the solid phase.

In an embodiment the aqueous medium is a spent fermentation broth obtained after producing the cellulase in *Trichoderma reesei.*

In an embodiment the cellulase harvested in the solid phase is dried. Suitable methods for drying the cellulase include spray drying, lyophilisation, evaporation, vacuum drying and drum drying. The cellulase in the solid phase may be washed before drying.

In an embodiment the cellulase solubility is controlled by adjusting chemical and physical conditions and/or protein concentration. The protein concentration may be controlled such that crystallization does not initiate until pH is adjusted. When enzyme concentration in the aqueous medium is increased above solubility of the enzyme in the conditions according to the first aspect, the enzyme starts to crystallize.

In an embodiment controlled crystallization can be initiated with seeding and polyethylene glycol addition at an acidic pH. Enzyme concentration in the aqueous medium is increased max. 2 times above the solubility limit at certain chemical and physical conditions, such as pH. Crystal yield over 80 % is achieved when the enzyme concentration is increased 6 times above its solubility limit at certain chemical and physical conditions. In an embodiment this yield is increased to over 95 % by adding PEG without need to increase the enzyme concentration further.

In an embodiment of the first aspect the pH is adjusted such that the pH of the spent fermentation broth is preferably lower than the pl of the cellulase to be crystallized. In an embodiment the pH is adjusted about 0.5 - 2.0 pH units, such as 0.5, 0.6, 0.7, 0.8, 0.9, 1.0, 1.1, 1.2, 1.3, 1.4, 1.5, 1.6, 1.7, 1.8, 1.9 or 2.0 pH units, lower than the pl of the cellulase to be crystallized at the beginning of crystallization. After the crystallization process has initiated, the pH can be raised higher, even above pl of the cellulase to be crystallized.

In an embodiment polyethylene glycol is added before or after adjusting pH. In another embodiment polyethylene glycol is added after adjusting pH and crystallization start, which is particularly advantageous because it improves yield of cellulase in crystalline form. Polyethylene glycol may be added before or after equilibrium is reached after pH adjustment.

In an embodiment polyethylene glycol is added sufficiently to increase the cellulase yield in a solid phase, which can be determined by selecting an amount of addition which provides an improved cellulase yield in a solid phase compared to the cellulase yield in a solid phase obtained using another amount. Suitable amounts of added polyethylene glycol are between 0.1 and 25% w/v, preferably between 0.1 and 10% w/v. In yet another embodiment polyethylene glycol is added to at least 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, or 10 % w/v.

In an embodiment the method further comprises: i. allowing transfer of the cellulase into the solid phase after step b.; and ii. adding polyethylene glycol after step i. Thus, step ii. is carried out after transfer of the cellulase into the solid phase has started. This can be monitored e.g. by visual inspection. This embodiment has the advantage of improving cellulase yield in the solid phase.

Suitable polyethylene glycols according to the invention have a molecular weight from about 1000 to about 80000 daltons, such as from about 2000 to about 10000 daltons, such as 2000, 2100, 2200, 2300, 2400, 2500, 2600, 2700, 2800, 2900, 3000, 3100, 3200, 3300, 3350, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, 9900 or 10000 daltons.

In an embodiment the method comprises adjusting at least one parameter selected from polyethylene glycol concentration, protein concentration, pH and temperature such that an improved, or a maximum, cellulase yield is achieved.

In an embodiment the cellulase is concentrated before adjusting pH to obtain a dry matter content selected from a range between 10 and 20 % w/v. This provides a favorable protein concentration to initiate crystallization. In another embodiment the dry matter content is increased to 10, 11, 12, 13, 14, 15, 16, 17, 18, 19 or 20 % w/v before adjusting pH. Dry matter content is a convenient parameter for following protein concentration of the spent fermentation broth from which the cellulase is harvested in cases where the spent fermentation broth contains a complex mixture of proteins, enzymes, carbohydrates and other macromolecules, for example in the case of fermentation medium. Dry matter content may be determined by methods known in the art.

In an embodiment the cellulase to be cystallised is not highly concentrated. In an embodiment the cellulase concentration is not more than 100g/l, such as about 100, 90 80, 70, 60, 50, 40, 30, 20 or 10g/l.

In another embodiment the cellulase is concentrated before adjusting pH and before adding polyethylene glycol. The cellulase concentration may be kept below supersaturation limit of the cellulase before pH adjustment. To further improve cellulase crystal yield, optimizing of all parameters including pH, protein concentration, polyethylene glycol concentration and temperature should preferably be done at the same time.

In an embodiment the cellulase comprises a cellulose binding moiety, preferably a fungal family 1 carbohydrate binding moiety.

The method according to the first aspect can be applied to concentrate the cellulase from any aqueous medium. Thus, the cellulase can be first produced, purified, and/or concentrated in a first aqueous medium, and then concentrated from the first or a further aqueous medium by using the method according to the first aspect.

In an embodiment the aqueous medium is a spent fermentation broth, preferably fermentation broth which is clarified before concentrating. The purpose of the clarification is to remove particulate matter such as cell residues. This further helps to achieve a purified cellulase product which is easy to handle, transport and store.

In an embodiment cellulase seed crystals are added to the spent fermentation broth, i.e. seeded. Seeding by this method has the advantage of faster initiation of crystallization.

In an embodiment the seed crystals are homogeneous nucleants preferably produced using the method according to the first aspect. Various seeding techniques utilizing either homogeneous or heterogeneous nucleants are known in the art (Bergfors 2007).

In an embodiment the cellulase is an endoglucanase, preferably a Glycoside hydrolase family 45 endoglucanase. These endoglucanases are particularly advantageous because they can be used in industrial applications requiring cellulase activity and they can be concentrated by the present method from the fermentation broth, at a very high purity, yield and stability.

In an embodiment the crystals are harvested after achieving solubility equilibrium between crystalline and soluble enzyme in the fermentation broth.

In an embodiment the crystals are washed when harvested. The washing can be carried out using a buffer essentially free of polyethylene glycol.

In an embodiment the spent fermentation broth is a clarified and concentrated fermentation broth whose pH is adjusted, optionally in two or more steps, and after pH adjustment polyethylene glycol is added.

In an embodiment the pH adjustment comprises adjusting the pH in two or more steps towards a target pH value. The target pH value may be a pH value selected from the range between pH 3 and 6. The pH adjustment may comprise waiting until an equilibrium is reached before the next pH adjustment step is started.

In an embodiment of the second aspect the composition comprises cellulase from washed crystals produced according to the first aspect.

In an embodiment the crystals are washed with a buffer essentially free of polyethylene glycol.

In an embodiment is provided a composition comprising at least partially crystallised cellulase produced according to an above aspect or embodiment, wherein the composition is in a liquid or in a solid form.

### EXAMPLES

The following examples are provided to illustrate various aspects of the present invention. They are not intended to limit the invention, which is defined by the accompanying claims.

### EXAMPLE 1 - Ma_Cel45A+CBM enzyme materials

Enzyme materials used for batch crystallizations were either clarified fermentation broths or concentrates of them, containing preservative to prevent microbial contamination, from several different fermentations of Ma_Cel45A+CBM protein. Fermentations were performed at pH 4.4 using two different complex cellulase-inducing mediums, medium 1 and 2. Fermentation broths were clarified by filtration and concentrated with 10 kDa ultrafiltration membrane to increase protein concentration. Enzyme activity of the protein was measured as the release of reducing sugars from carboxymethylcellulose (3 % (w/v) CMC) at 50°C in 50 mM Hepes buffer pH 7.0 in 10 min (NCU activity, nkat; Bailey and Nevalainen, 1981; Haakana et al., 2004).

### EXAMPLE 2 - Batch crystallizations of Ma_Cel45A+CBM protein

Concentrate used in batch crystallization was prepared from fermentation made with the medium 2. Impure enzyme concentrate solution was stored at + 4 °C. Crystallization of the enzyme, Ma_Cel45A+CBM, started at decreased pH 4.1. Calculated crystal yield of this batch was 40 % analyzed based on soluble activity in the mother liquor.

### EXAMPLE 3 - The effect of pH on the solubility of crystals of Ma_Cel45A+CBM protein

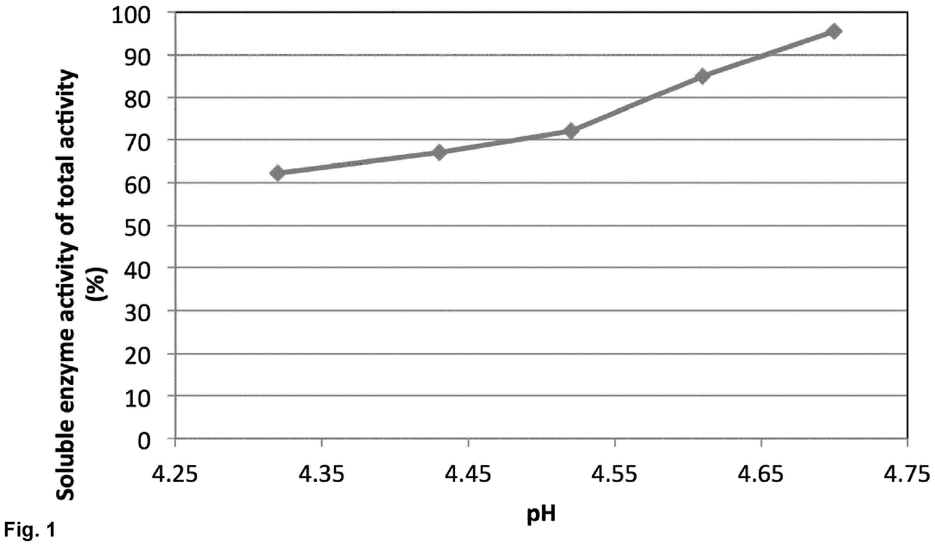

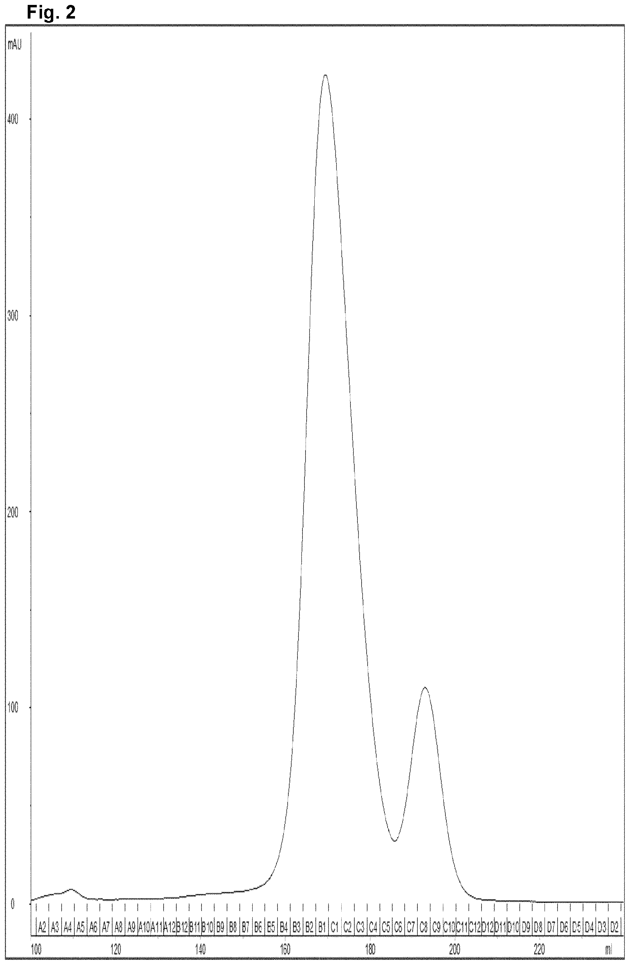

In order to study effect of pH on the solubility of crystals of Ma_Cel45A+CBM protein 50 ml of crystal slurry prepared in example 2 was taken apart. pH of this slurry was adjusted to pH 6.19 with 2 M NaOH. Crystals dissolved after pH adjusting. For solubility tests part of crystal slurry prepared in example 2 was mixed with dissolved crystals to receive pH series. Experiments were made in 15 ml tubes and total volumes of the experiment were 10 ml. The tubes were shaken gently on vertical shaker. The tubes were incubated at + 4 °C for at least two days to achieve solubility equilibrium. The resulting soluble enzyme activities and pH were determined. The results are represented in table 1 and illustrated graphically in figure 1. The gel filtration result of washed crystals of Ma_Cel45A+CBM protein shows high purity as illustrated in figure 2.

**Table 1. Effect of pH on the solubility of crystals of Ma_Cel45A+CBM protein.**

| Crystal slurry pH 4.13 (ml) | Crystal slurry pH 6.19 (ml) | pH | Soluble enzyme activity of total activity (%) |
|---|---|---|---|
| 10 | 0 | 4.32 | 62.1 |
| 9 | 1 | 4.43 | 67.1 |
| 8 | 2 | 4.52 | 72.0 |
| 7 | 3 | 4.61 | 84.8 |
| 6 | 4 | 4.70 | 95.5 |

### EXAMPLE 4 - Batch crystallizations of Ma_Cel45A+CBM protein

Concentrate used in batch crystallization was prepared from fermentation made with the medium 1. Impure enzyme concentrate solution was stored at + 4 °C. Crystallization was initiated by adjusting pH of the concentrate to 4.4. Later on crystal yield was improved by adjusting pH to 4.0. Crystal amount increasing was seen clearly by visual observation. Calculated crystal yield of this batch was 80 % analysed based on soluble activity in the mother liquor.

### EXAMPLE 5 - Preparation of crystal suspensions for solubility experiments

Crystal slurry from batch crystallization prepared in example 4 was centrifuged to separate crystals and mother liquid. Half volume of supernatant was removed to concentrate the slurry. Crystals and remaining supernatant were pooled together to get uniform crystal slurry. Experiments were made in 2 ml Eppendorf tubes. 1.6 ml of crystal slurry and 0.4 ml of reagent solution were mixed in the tubes. These samples were divided into two 2 ml Eppendorf tubes, one for total sample and one for soluble sample, 1 ml per tube. The tubes were shaken gently on vertical shaker. The tubes were incubated at + 4 °C for at least two days to achieve solubility equilibrium.

### EXAMPLE 6 - The effect of pH on the solubility of crystals of Ma_Cel45A+CBM protein

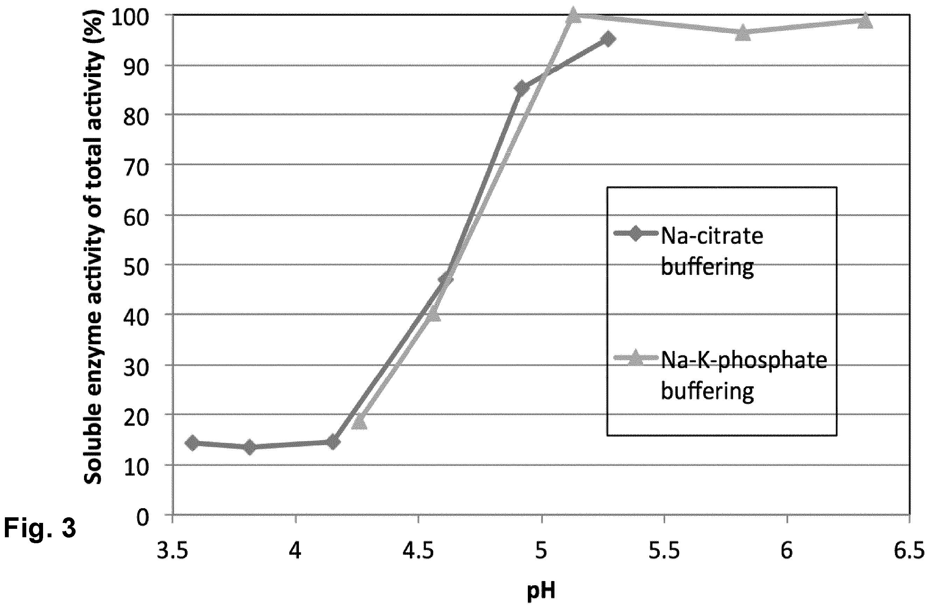

Using the method in example 5, a series of solubility experiments were performed at + 4 °C in a reagent solution containing 0.1 M Na-citrate buffer (nominal pH 3.2-6.2) or 0.1 M Na-K-phosphate buffer (nominal pH 5.6-7.5). The crystals of Ma_Cel45A+CBM protein were allowed to achieve solubility equilibrium in the various reagent solutions, and the resulting soluble enzyme activities and pH were determined. The results are represented in table 2 and illustrated graphically in figure 3.

**Table 2. Effect of pH on the solubility of crystals of Ma_Cel45A+CBM protein.**

| Na-citrate (mM) | Na-K-phosphate (mM) | pH | Soluble enzyme activity of total activity (%) |
|---|---|---|---|
| 100 | | 3.58 | 14 |
| 100 | | 3.81 | 13 |
| 100 | | 4.15 | 15 |
| 100 | | 4.61 | 47 |
| 100 | | 4.92 | 85 |
| 100 | | 5.27 | 95 |
| | 100 | 4.26 | 19 |
| | 100 | 4.56 | 40 |
| | 100 | 5.13 | 100 |
| | 100 | 5.82 | 97 |
| | 100 | 6.32 | 99 |

### EXAMPLE 7 - The effect of pH on the solubility of crystals of Ma_Cel45A+CBM protein in the presence of PEG 3350

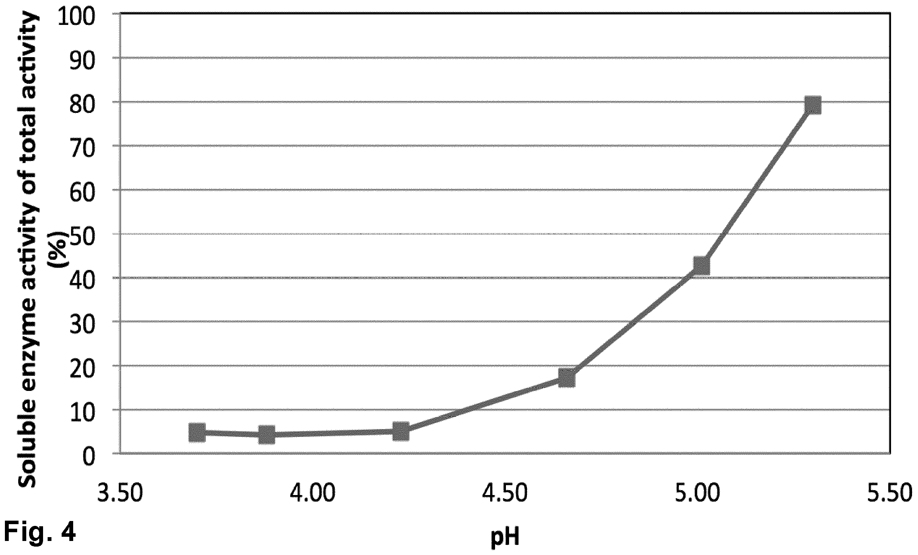

Using the method in example 5, a series of solubility experiments were performed at + 4 °C in a reagent solution containing 0.1 M Na-citrate (nominal pH 3.2-6.2) and 5 % (w/v) PEG 3350. The crystals of Ma_Cel45A+CBM protein were allowed to achieve solubility equilibrium in the various reagent solutions, and the resulting soluble enzyme activities and pH were determined. The results are represented in table 3 and illustrated graphically in figure 4. These results show clearly strong solubility decreasing effect of PEG compared to the solubility data achieved without PEG (the example 6).

**Table 3. Effect of pH on the solubility of crystals of Ma_Cel45A+CBM protein in the presence of 5 % (w/v) PEG 3350.**

| PEG 3350 (%, w/v) | Na-citrate (mM) | pH | Soluble enzyme activity of total activity (%) | Comments |
|---|---|---|---|---|
| 5 | 100 | 3.70 | 5 | opal |
| 5 | 100 | 3.88 | 4 | opal |
| 5 | 100 | 4.23 | 5 | opal |
| 5 | 100 | 4.66 | 17 | opal |
| 5 | 100 | 5.01 | 43 | little opal |
| 5 | 100 | 5.30 | 79 | slightly opal |

### EXAMPLE 8 - The effect of PEG 8000 concentrations on the solubility of crystals of Ma_Cel45A+CBM protein

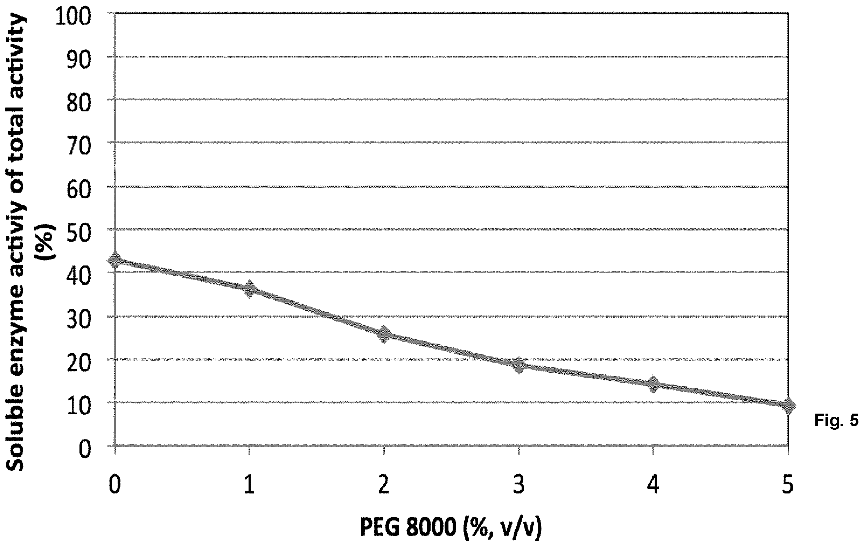

Using the method in example 5, a series of solubility experiments were performed at + 4 °C in a reagent solution containing 0.1 M Na-citrate (nominal pH 5.0) and 0-5 % (w/v) PEG 8000. The crystals of Ma_Cel45A+CBM protein were allowed to achieve solubility equilibrium in the various reagent solutions, and the resulting soluble enzyme activities and pH were determined. The results are represented in table 4 and illustrated graphically in figure 5. These results show clearly that PEGs of different molecular weights also work in crystallization.

**Table 4. Effect of PEG 8000 concentrations on the solubility of crystals of Ma_Cel45A+CBM protein.**

| PEG 8000 (%, w/v) | Na-citrate (mM) | pH | Soluble enzyme activity of total activity (%) | Comments |
|---|---|---|---|---|
| 0 | 100 | 4.59 | 43 | opal |
| 1 | 100 | 4.60 | 36 | opal |
| 2 | 100 | 4.61 | 26 | opal |
| 3 | 100 | 4.63 | 19 | opal |
| 4 | 100 | 4.67 | 14 | opal |
| 5 | 100 | 4.66 | 9 | opal |

### EXAMPLE 9 - Batch crystallizations of Ma_Cel45A+CBM protein with PEG 8000

Concentrate used in batch crystallizations was prepared from fermentation made with medium 1. Impure enzyme concentrate solution was stored at + 4 °C. After one day storage pH was adjusted with 2 M H₂SO₄ from pH 5.15 to pH 4.51. After 3 days storage pH was adjusted with 2 M H₂SO₄ from pH 4.57 to pH 4.22. After pH adjusting batch was divided and for two 40% (w/w) PEG 8000 was added to get the final PEG concentrations shown in table 5. Crystallizations were let to continue for one day. The results are represented in the table 5. These results show clearly strong crystal yield improving effect of low concentrations of PEG.

**Table 5. Activity yields of batch crystallizations without and with low concentrations of PEG.**

| PEG 8000 (%, w/w) | Activity yield (%) in crystals |
|---|---|
| 0 | 30 |
| 0.94 | 47 |
| 2.90 | 80 |

### EXAMPLE 10 - Preparation of washed crystal suspension for solubility experiments

Crystal slurry from batch crystallization prepared in example 9 was centrifuged to separate crystals and mother liquid. Crystals were washed twice with pH 4.16 Na-citrate buffer containing preservative, and without polyethylene glycol. Washed crystals were pooled to the washing buffer. Experiments were made in 2 ml Eppendorf tubes. 1.5 ml of washed crystal slurry and 0.4 ml of reagent solution were mixed in the tubes. These samples were divided into two 2 ml Eppendorf tubes, one for total sample and one for soluble sample, 1 ml per tube. The tubes were shaken gently on vertical shaker. The tubes were incubated at + 4 °C for at least two days to achieve solubility equilibrium.

### EXAMPLE 11 - The effect of PEG 8000 concentration on the solubility of washed crystals of Ma_Cel45A+CBM protein

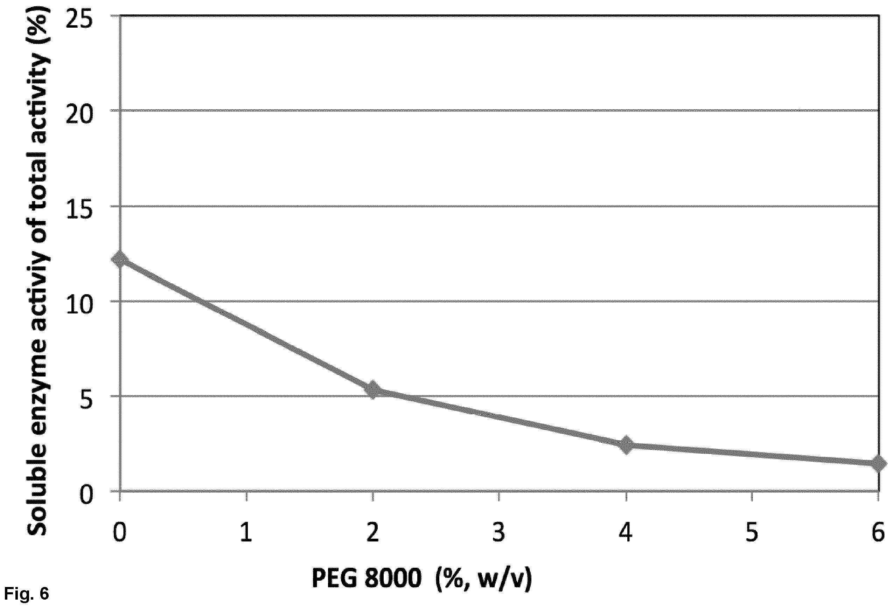

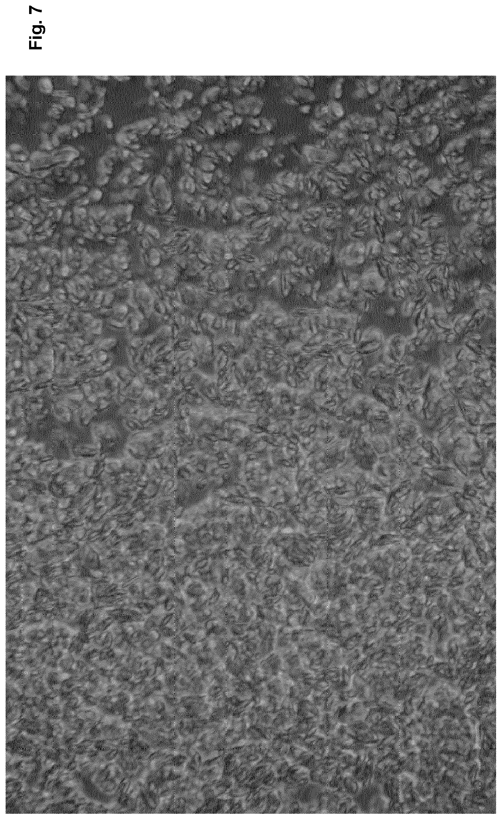

Using the method in example 10, a series of solubility experiments were performed at + 4 °C in a reagent solution containing 0-6 % (w/v) PEG 8000. The crystals were allowed to achieve solubility equilibrium in the various reagent solutions, and the resulting soluble enzyme activities and pH were determined. The results are represented in table 6 and illustrated graphically in figure 6. Crystals formed with 6 % (w/v) PEG 8000 are shown in figure 7. These results show decreasing enzyme solubility by increasing PEG concentration.

**Table 6. Effect of PEG 8000 concentration on the solubility of washed crystals of Ma_Cel45A+CBM protein.**

| PEG 8000 (%, w/v) | pH | Soluble enzyme activity of total activity (%) |
|---|---|---|
| 0 | 4.22 | 12 |
| 2 | 4.21 | 5 |
| 4 | 4.25 | 2 |
| 6 | 4.28 | 1 |

### EXAMPLE 12 - The effect of ammonium sulphate concentration on the solubility of washed crystals of Ma_Cel45A+CBM protein

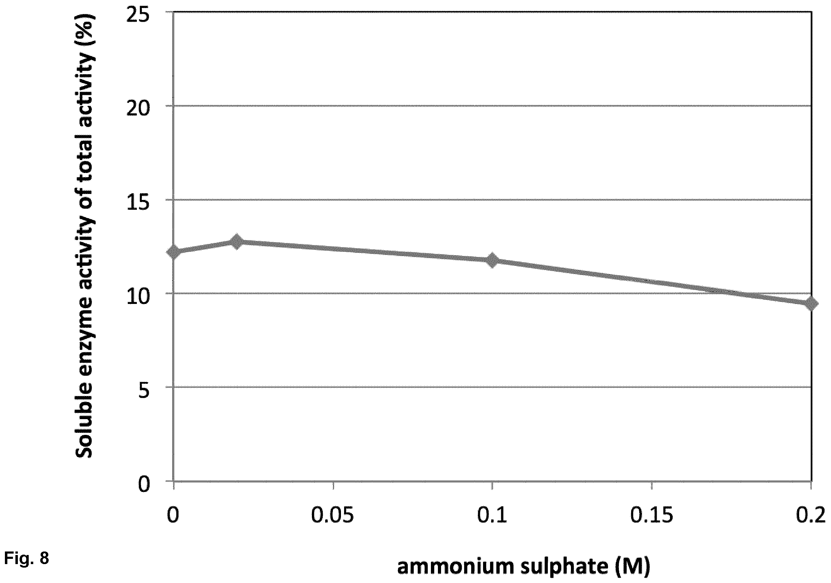

The effect of ammonium sulphate as commonly used protein precipitant was studied here. Using the method in example 10, a series of solubility experiments were performed at + 4 °C in a reagent solution containing 0-200 mM ammonium sulphate. The crystals were allowed to achieve solubility equilibrium in the various reagent solutions, and the resulting soluble enzyme activities and pH were determined. The results are represented in table 7 and illustrated graphically in figure 8. These results show that ammonium sulphate has not similar enzyme solubility decreasing effect as PEG has.

**Table 7. Effect of ammonium sulphate concentration on the solubility of washed crystals of Ma_Cel45A+CBM protein.**

| Ammonium sulphate (M) | pH | Soluble enzyme activity of total activity (%) |
|---|---|---|
| 0 | 4.22 | 12 |
| 0.02 | 4.22 | 13 |
| 0.1 | 4.17 | 12 |
| 0.2 | 4.13 | 9 |

### EXAMPLE 13 - The effect of PEG 8000 when added to the solution of washed crystals of Ma_Cel45A+CBM protein after dissolving at pH 5

Using the method in example 10 in exception that the pH of the crystal slurry was adjusted at first to 4.84 to dissolve the crystals, a series of crystallization experiments were performed at + 4 °C in a reagent solution containing 0-10 % (w/v) PEG 8000. The experiments were allowed to achieve solubility equilibrium in the various reagent solutions. The results are represented in table 8. This data shows importance of order of method steps on final outcome. At pH around 5 (close to theoretical pl of the enzyme), PEG addition (here 6 % or higher) results in precipitation instead of crystallization.

Thus, in an embodiment the PEG can be removed e.g. by washing or a similar method to further improve stability of the crystalline cellulase and avoid precipitation.

**Table 8. Results of the experiments when PEG 8000 added at pH 5.**

| PEG 8000 (%, w/v) | Comments |
|---|---|
| 0 | clear |
| 2 | clear |
| 6 | strong precipitation |
| 10 | strong precipitation |

### EXAMPLE 14 - The effect of pH on the solubility of washed crystals of Ma_Cel45A+CBM protein in the presence of PEG 8000

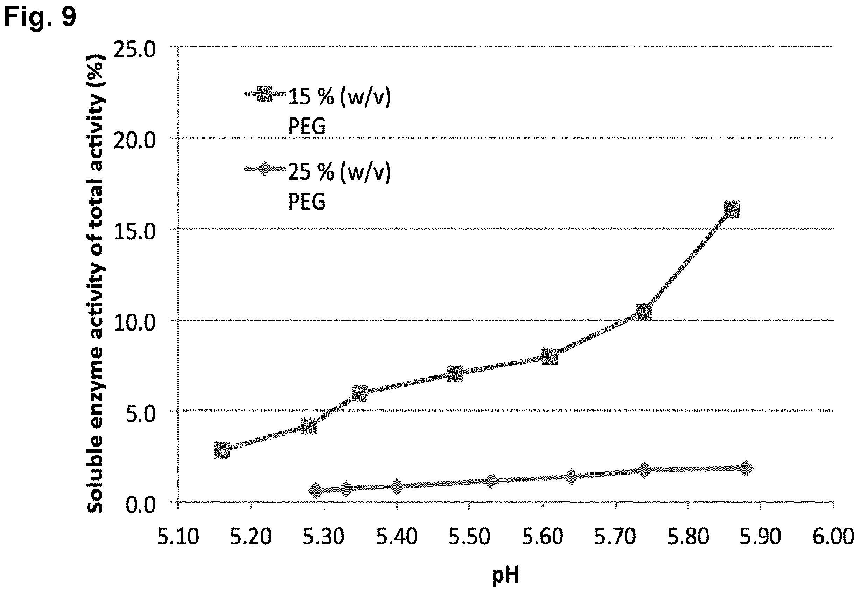

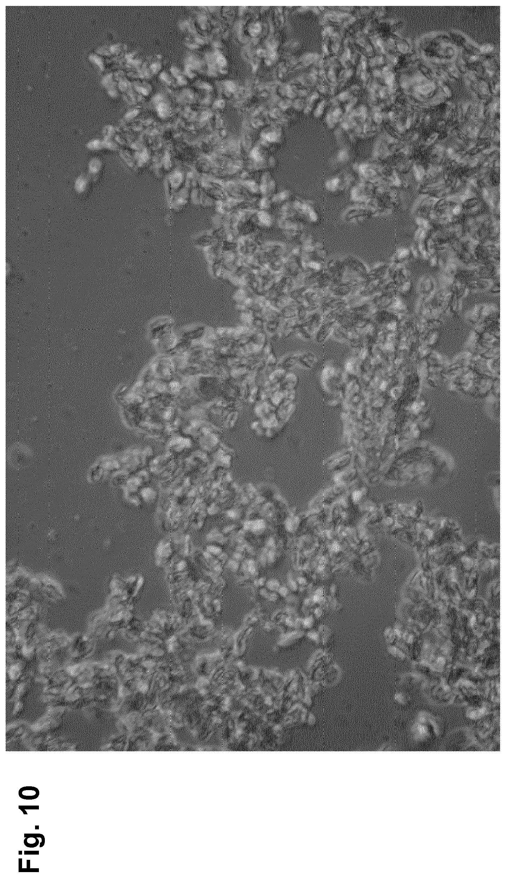

Using the method in example 10, a series of solubility experiments were performed at + 4 °C in a reagent solution containing 15 % or 25 % (w/v) PEG 8000. pH values between 5.16 and 5.88 were achieved by using different ratios of 50 mM Na-citrate buffers at nominal pH 4.6 and 6.2 in the solutions. The crystals were allowed to achieve solubility equilibrium in the various reagent solutions, and the resulting soluble enzyme activities and pH were determined. The results are represented in table 9 and illustrated graphically in figure 9. Crystals in 25 % (w/v) PEG 8000, 50 mM Na-citrate at pH 5.88 are shown in figure 10. This data shows that solubility of the enzyme at pH range 5.2 - 5.9 (above theoretical pl of the enzyme), can be controlled by maintaining high PEG concentration.

**Table 9. Effect of pH on the solubility of washed crystals of Ma_Cel45A+CBM protein in the presence of 15 % or 25 % (w/v) PEG 8000.**

| 15 % (w/v) PEG: pH | Soluble enzyme activity of total activity (%) | Comments |
|---|---|---|
| 5.16 | 2.8 | white crystal slurry |
| 5.28 | 4.2 | white crystal slurry |
| 5.35 | 6.0 | white crystal slurry |
| 5.48 | 7.0 | white crystal slurry |
| 5.61 | 8.0 | white crystal slurry |
| 5.74 | 10.5 | white crystal slurry |
| 5.86 | 16.1 | white crystal slurry |

| 25 % (w/v) PEG: pH | Soluble enzyme activity of total activity (%) | Comments |
|---|---|---|
| 5.29 | 0.6 | white crystal slurry, lots of irregular granular crystals (confirmed by microscopy) |
| 5.33 | 0.7 | white crystal slurry |
| 5.40 | 0.9 | white crystal slurry |
| 5.53 | 1.2 | white crystal slurry |
| 5.64 | 1.4 | white crystal slurry |
| 5.74 | 1.7 | white crystal slurry |
| 5.88 | 1.8 | white crystal slurry, lots of irregular granular crystals (confirmed by microscopy) |

### EXAMPLE 15 - The effect of temperature on the solubility of washed crystals of Ma_Cel45A+CBM protein in the presence of PEG 8000

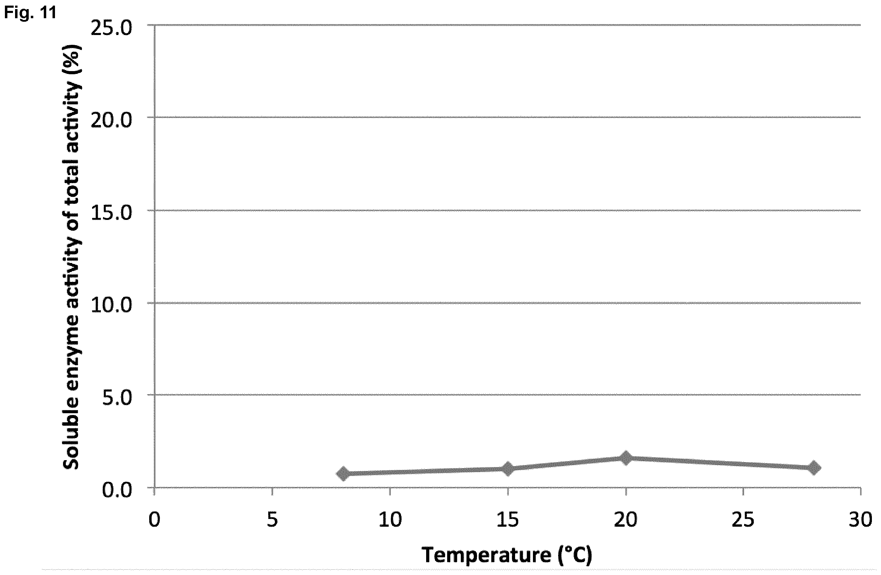

Using the method in example 10, a series of solubility experiments were performed at + 8°C, + 15°C, + 20°C and + 28°C in a reagent solution at pH 4.16 containing 50 mM Na-citrate and 10 % (w/v) PEG 8000. The crystals were allowed to achieve solubility equilibrium at these temperatures for 2 days, and the resulting soluble enzyme activities were determined. The results are represented in table 10 and illustrated graphically in figure 11. This data shows that effect of temperature on enzyme solubility is quite low at studied chemical conditions.

**Table 10. Effect of temperature on the solubility of washed crystals of Ma_Cel45A+CBM protein in the presence of 10 % (w/v) PEG 8000.**

| Temperature (°C) | Soluble enzyme activity of total activity (%) | Comments |
|---|---|---|
| 8 | 0.7 | white crystal slurry, lots of irregular granular crystals (confirmed by microscopy) |
| 15 | 1.0 | white crystal slurry |
| 20 | 1.6 | white crystal slurry |
| 28 | 1.1 | white crystal slurry, lots of irregular granular crystals (confirmed by microscopy) |

### EXAMPLE 16 - Batch crystallization of Ma_Cel45A+CBM protein

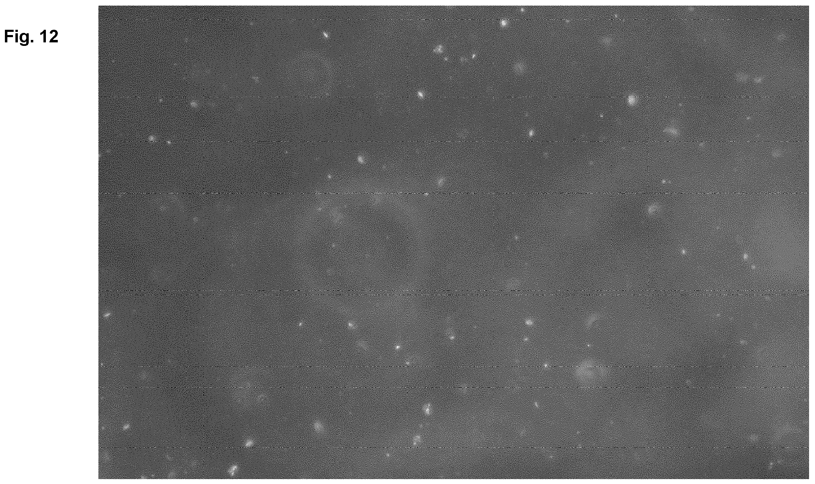

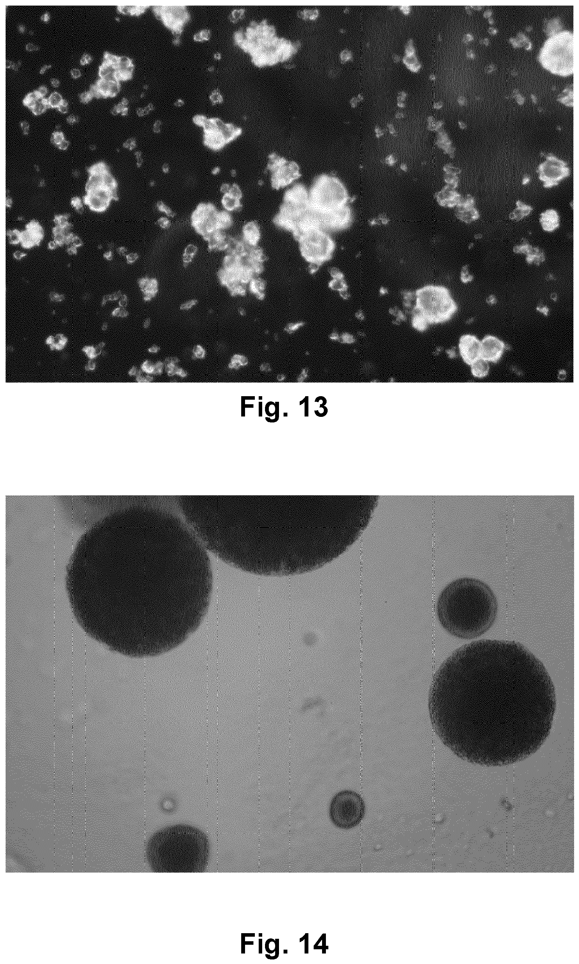

Fermentation of Ma_Cel45A+CBM protein was performed at pH 4.4 using complex cellulose-inducing medium 1. At the end of fermentation small sample of solid free, clarified fermentation broth was frozen. After thawing and sterile filtering this sample was crystallized as follows. pH of the sample was adjusted to 3.42 by adding 100 mM Na-citrate buffer at nominal pH 3.2. The sample was incubated overnight at +8°C but it stayed clear. Next day the sample was seeded. 2 µl of seed crystals from earlier crystallized batch was added to approximately 1.7 ml of this sample. Incubation was continued for two days at +8°C but clear progress of crystallization was not seen even if the seed crystals were noticed in the liquid by microscopy (figure 12). Incubation was continued overnight at +4°C under agitation but clear progress of crystallization was not seen. Next day aqueous solution of PEG 8000 was added to the sample to reach final concentration 3 % (w/v) and incubation was continued overnight at +4°C under agitation. Next day good progress of crystallization was noticed in the liquid by microscopy (figure 13). This result indicates importance of PEG addition for enzyme crystallization from fermentation broth without concentration step.

### EXAMPLE 17 - Screening of crystallization conditions for At_Cel45A+CBM protein

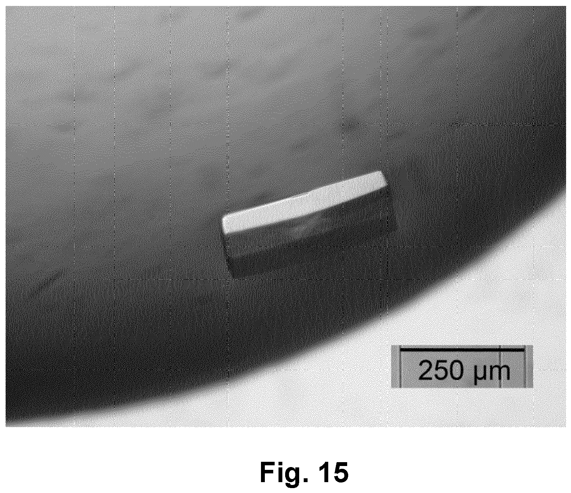

Screening of crystallization conditions for At_Cel45A+CBM protein was done with hanging drops which is a traditional method used in protein crystal screening. Concentrated fermentation broth was used as a protein sample. Semicrystalline spherulites shown in figure 14 were achieved using 5 % (w/v) PEG 4000 as precipitant in Ca-acetate buffer at pH 5.6. Core domain of At_Cel45A without CBM forms well-ordered crystals at similar PEG 8000 containing conditions as shown in figure 15.

The foregoing description has provided, by way of non-limiting examples of particular implementations and embodiments of the invention, a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed embodiments of this invention may be used to advantage without the corresponding use of other features. As such, the foregoing description shall be considered as merely illustrative of the principles of the present invention, and not in limitation thereof. Hence, the scope of the invention is only restricted by the appended patent claims.

## Claims

1. A method for concentrating a cellulase from spent fermentation broth, the method comprising the sequence of steps:
a. providing the cellulase in the spent fermentation broth in a soluble form;
b. controlling cellulase solubility by adjusting protein concentration;
adjusting the pH value to a pH value selected between pH 3 and 6 to provide and maintain the cellulase in a solid phase, and adding polyethylene glycol to increase the amount of the cellulase in the solid phase; and
c. harvesting the cellulase in the solid phase.

2. The method of claim 1 comprising adjusting at least one parameter selected from polyethylene glycol concentration, protein concentration, and temperature.

3. The method of any one of claims 1-2 wherein the cellulase is concentrated before adjusting pH to obtain a dry matter content selected from a range between 10 and 20 % w/v.

4. The method of any one of claims 1-3 wherein the cellulase comprises a cellulose binding moiety, preferably a fungal family 1 carbohydrate binding moiety.

5. The method of any one of claims 1-4 wherein the spent fermentation broth is clarified before concentrating.

6. The method of any one of claims 1-5 wherein cellulase seed crystals are added to the spent fermentation broth.

7. The method of claim 6 wherein the seed crystals are produced using the method of any one of claims 1-5.

8. The method of any one of claims 1-7 wherein the cellulase is an endoglucanase, preferably a Glycoside hydrolase family 45 endoglucanase.

9. The method of any one of claims 1-8 wherein polyethylene glycol is added before or after equilibrium is reached after adjusting the pH.

10. The method of claim 9 wherein in pH adjustment comprises adjusting the pH in two or more steps towards a target pH value.

11. The method of any one of claims 1-10 wherein polyethylene glycol is added to a concentration selected between 0.1 and 25 % w/v.

12. The method of any one of claims 1-11 wherein the cellulase in the solid phase is harvested in the form of crystals, and the crystals are washed.

13. A composition comprising at least partially crystallised cellulase produced according to any one of claims 1-12, wherein the composition is in a liquid or in a solid form.

14. A composition obtained by drying the cellulase produced according to any one of claims 1-12 or the composition according to the claim 13.

15. Use of the composition of claim 13 or 14 to modify cellulose in biomass processing, biofuel, starch, textile, detergent, pulp and paper, food, baking, feed or beverage industry.

## Patentansprüche

1. Verfahren zum Konzentrieren einer Cellulase aus verbrauchter Fermentationsbrühe, wobei das Verfahren nacheinander die folgenden Schritte umfasst:
a. Bereitstellen der Cellulase in der verbrauchten Fermentationsbrühe in löslicher Form;
b. Steuern der Cellulase-Löslichkeit durch Einstellen der Proteinkonzentration;
Einstellen des pH-Wertes auf einen zwischen pH 3 und 6 gewählten pH-Wert, um die Cellulase in einer festen Phase bereitzustellen und zu erhalten, und Zugeben von Polyethylenglykol, um die Menge der Cellulase in der festen Phase zu erhöhen; und
c. Gewinnen der Cellulase in der festen Phase.

2. Verfahren nach Anspruch 1, umfassend das Einstellen mindestens eines Parameters, ausgewählt aus Polyethylenglykol-Konzentration, Protein-Konzentration und Temperatur.

3. Verfahren nach einem der Ansprüche 1-2, wobei die Cellulase vor der pH-Einstellung konzentriert wird, um einen Trockensubstanzgehalt zu erhalten, ausgewählt aus einem Bereich zwischen 10 und 20 % w/v.

4. Verfahren nach einem der Ansprüche 1-3, wobei die Cellulase eine Cellulose-bindende Einheit, vorzugsweise eine Kohlenhydrat-bindende Einheit der Pilzfamilie 1, umfasst.

5. Verfahren nach einem der Ansprüche 1-4, wobei die verbrauchte Fermentationsbrühe vor dem Konzentrieren geklärt wird.

6. Verfahren nach einem der Ansprüche 1-5, wobei der verbrauchten Fermentationsbrühe Cellulase-Impfkristalle zugesetzt werden.

7. Verfahren nach Anspruch 6, wobei die Impfkristalle unter Verwendung des Verfahrens nach einem der Ansprüche 1-5 hergestellt werden.

8. Verfahren nach einem der Ansprüche 1-7, wobei die Cellulase eine Endoglucanase ist, vorzugsweise eine Endoglucanase der Glycosid-Hydrolase-Familie 45.

9. Verfahren nach einem der Ansprüche 1-8, wobei Polyethylenglykol vor oder nach Erreichen des Gleichgewichts nach Einstellung des pH-Wertes zugegeben wird.

10. Verfahren nach Anspruch 9, wobei die pH-Einstellung das Einstellen des pH-Wertes in zwei oder mehr Schritten in Richtung eines Ziel-pH-Werts umfasst.

11. Verfahren nach einem der Ansprüche 1-10, wobei Polyethylenglykol in einer Konzentration zwischen 0,1 und 25 % w/v zugegeben wird.

12. Verfahren nach einem der Ansprüche 1-11, wobei die Cellulase in der festen Phase in Form von Kristallen gewonnen wird und die Kristalle gewaschen werden.

13. Zusammensetzung, umfassend mindestens teilweise kristallisierte Cellulase, hergestellt nach einem der Ansprüche 1-12, wobei die Zusammensetzung in flüssiger oder fester Form vorliegt.

14. Zusammensetzung, erhalten durch Trocknen der nach einem der Ansprüche 1-12 hergestellten Cellulase oder der Zusammensetzung nach Anspruch 13.

15. Verwendung der Zusammensetzung nach Anspruch 13 oder 14 zum Modifizieren von Cellulose in der Biomasse-Verarbeitung, Biokraftstoff-, Stärke-, Textil-, Waschmittel-, Zellstoff- und Papier-, Lebensmittel-, Back-, Futtermittel- oder Getränkeindustrie.

## Revendications

1. Un procédé pour concentrer une cellulase à partir d'un jus de fermentation usagé, le procédé comprenant la séquence d'étapes suivante :
a. fournir la cellulase dans le jus de fermentation usagé sous une forme soluble ;
b. contrôler la solubilité de la cellulase en ajustant la concentration des protéines ;
ajuster la valeur du pH à une valeur de pH choisie entre pH 3 et 6 pour fournir et maintenir la cellulase en phase solide, et ajouter du polyéthylène glycol pour augmenter la quantité de cellulase dans la phase solide ; et
c. récolter la cellulase dans la phase solide.

2. Le procédé selon la revendication 1, comprenant le fait d'ajuster au moins un paramètre choisi parmi la concentration en polyéthylène glycol, la concentration en protéines et la température.

3. Le procédé selon l'une quelconque des revendications 1 et 2, dans laquelle la cellulase est concentrée avant le fait d'ajuster le pH pour obtenir une teneur en matière sèche choisie dans une gamme allant de 10 et 20 % p/v.

4. Le procédé selon l'une quelconque des revendications 1 à 3, dans lequel la cellulase comprend un fragment de liaison à la cellulose, de préférence un fragment de liaison aux hydrates de carbone de la famille fongique 1.

5. Le procédé selon l'une quelconque des revendications 1 à 4, dans laquelle le jus de fermentation usagé est clarifié avant la concentration.

6. Le procédé selon l'une quelconque des revendications 1 à 5, dans laquelle des cristaux d'ensemencement de cellulase sont ajoutés au jus de fermentation usagé.

7. Le procédé selon la revendication 6, dans laquelle les cristaux d'ensemencement sont produits en utilisant le procédé selon l'une quelconque des revendications 1 à 5.

8. Le procédé selon l'une quelconque des revendications 1 à 7, dans lequel la cellulase est une endoglucanase, de préférence une endoglucanase de la famille 45 des hydrolases de glycoside.

9. Le procédé selon l'une des revendications 1 à 8, dans laquelle du polyéthylène glycol est ajouté avant ou après que l'équilibre soit atteint après l'ajustement du pH.

10. Le procédé selon la revendication 9, dans laquelle l'ajustement du pH comprend le fait d'ajuster le pH en deux ou plusieurs étapes vers une valeur de pH cible.

11. Le procédé selon l'une quelconque des revendications 1 à 10, dans laquelle du polyéthylène glycol est ajouté à une concentration choisie entre 0,1 et 25 % p/v.

12. Le procédé selon l'une des revendications 1 à 11, dans laquelle la cellulase en phase solide est récoltée sous forme de cristaux, et les cristaux sont lavés.

13. Une composition comprenant une cellulase au moins partiellement cristallisée produite selon l'une quelconque des revendications 1 à 12, dans laquelle la composition est sous une forme liquide ou solide.

14. Une composition obtenue par séchage de la cellulase produite selon l'une quelconque des revendications 1 à 12 ou de la composition selon la revendication 13.

15. Utilisation de la composition selon la revendication 13 ou la revendication 14 pour modifier la cellulose dans le traitement d'une biomasse, de biocarburants, de l'amidon, du textile, des détergents, de la pâte et du papier, des aliments, des produits de boulangerie, de l'industrie alimentaire ou des boissons.
